(19) **Europäisches Patentamt**
**European Patent Office**
**Office européen des brevets**

(11) **EP 4 465 871 B1**

(12) **EUROPEAN PATENT SPECIFICATION**

(45) Date of publication and mention
of the grant of the patent:
**17.06.2026   Bulletin 2026/25**

(21) Application number: **23700722.4**

(22) Date of filing: **11.01.2023**

(51) International Patent Classification (IPC):
***A61B 3/10*** *(2006.01)*

(52) Cooperative Patent Classification (CPC):
**A61B 3/102**

(86) International application number:
**PCT/EP2023/050514**

(87) International publication number:
**WO 2023/138963 (27.07.2023 Gazette 2023/30)**

(54) **METHODS AND SYSTEMS FOR CONFOCAL FUNCTION DETERMINATION AND CORRECTION IN AN OCT IMAGING SYSTEM**

VERFAHREN UND SYSTEME ZUR KONFOKALEN FUNKTIONSBESTIMMUNG UND -KORREKTUR IN EINEM OCT-BILDGEBUNGSSYSTEM

PROCÉDÉS ET SYSTÈMES DE DÉTERMINATION DE FONCTION CONFOCALE ET CORRECTION DANS UN SYSTÈME D'IMAGERIE OCT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:   **19.01.2022   EP 22152195**

(43) Date of publication of application:
**27.11.2024   Bulletin 2024/48**

(73) Proprietor: **Heidelberg Engineering GmbH**
**69115 Heidelberg (DE)**

(72) Inventors:
• **KÜBLER, Johannes**
  **68723 Schwetzingen (DE)**
• **FISCHER, Jörg**
  **69221 Dossenheim (DE)**
• **DE BOER, Johannes F.**
  **1186 DC Amstelveen (NL)**

(74) Representative: **Wesch, Arno**
**Patentanwaltskanzlei Dr. Arno Wesch**
**Augustaanlage 32**
**68165 Mannheim (DE)**

(56) References cited:
• **DWORK NICHOLAS ET AL: "Automatically Determining the Confocal Parameters From OCT B-Scans for Quantification of the Attenuation Coefficients", IEEE TRANSACTIONS ON MEDICAL IMAGING, IEEE, USA, vol. 38, no. 1, 1 January 2019 (2019-01-01), pages 261 - 268, XP011694960, ISSN: 0278-0062, [retrieved on 20181228], DOI: 10.1109/TMI.2018.2861570**
• **LIN HUANG ET AL: "Attenuation Coefficient of Tissue Based on Modified Depth-Resolved Model", 2021 IEEE INTERNATIONAL CONFERENCE ON MECHATRONICS AND AUTOMATION (ICMA), IEEE, 8 August 2021 (2021-08-08), pages 242 - 247, XP033963863, DOI: 10.1109/ICMA52036.2021.9512733**

**Description**

The invention is related to an OCT imaging system according to the preamble of claim 1.

Such an imaging system is disclosed by DWORKS NICHOLAS ET AL: "Automatically Determining the Confocal Parameters from OCT B-Scans for Quantification of the Attenuation Coefficients", IEE TRANSACTIONS IN MEDICAL IMAGING, IEEE, USA, vol. 38, no.1, 1 January 2019, p. 261-268, XP011694960, ISSN:0278-0062.

Optical coherence tomography (OCT) is an optical imaging technology for performing cross sectional imaging of tissue structures.

OCT measures the scattering profile of a sample along the OCT beam. Each scattering profile is called an A-line.

Two dimensional cross-sectional images, called B-scans are built up from many A-lines, with the OCT beam transversely scanning over the sample.

In time domain OCT (TD-OCT), the optical path length between the sample and reference arms needs to be mechanically scanned.

In Fourier domain OCT (FD-OCT), however, the optical path length difference between the sample and reference arm is not mechanically scanned.

Instead, a full A-line is obtained in parallel for all points along the axial line by Fourier transformation of the wavelength sweep of a swept source in swept source OCT (SS-OCT) or resolving the spectrum of a superluminescent diode (SLD) on a spectrally resolved and/ or line scan camera in spectral domain OCT (SD-OCT).

An A-line I(z) along z can be described by

$$I(z) \propto r(z)h(z)\alpha\mu_t \exp(-2\mu_t z)$$

$$(1.1)$$

with the roll-off $r(z)$, the confocal function $h(z)$, the backscattering fraction $\alpha$, and the attenuation coefficient $\mu_t$.

Up to date in clinics OCT technology is mainly used for imaging of tissue structures and measuring geometric parameters like layer thicknesses or distances between structures.

In addition, in OCTA the variance of speckle and/or phase is evaluated for assessing and displaying blood flow in vessels.

It is the object of the invention to overcome the drawbacks of the state of the art.

The object of the invention is achieved by means of the features of claim 1.

According to the invention it has been found, that because an OCT beam is focused onto a sample with a lens, the A-line scattering profile along z depends on the confocal function $h(z)$ of the imaging system, i.e., focus position $z_f$ and Rayleigh length $z_R$.

$$h(z) = \left[ \left( \frac{z - z_f}{z_R} \right)^2 + 1 \right]^{-1}$$

$$(1.2)$$

Then it has been found, that the influence of the confocal function needs to be corrected for if quantitative parameters such as the attenuation coefficient are to be determined from an A-line.

To determine the confocal function the Rayleigh length and focus of the optical system must be known. Especially in in-vivo human retinal imaging the sample is moving and accommodating complicating the determination of the focus location.

According to the invention, it has been found further, that to determine the confocal function, the common characteristics of two A-lines of the same sample location acquired with different focal positions can be exploited.

Taking the ratio of the two A-lines acquired with a focus offset allows for the determination of focus depth and Rayleigh length [1-4], e.g., by fitting the following equation to the ratio

$$\frac{I_1(z)}{I_2(z)} = \frac{h_1(z)}{h_2(z)} = \frac{\left(\dfrac{z - (z_{f1} + \Delta z_f)}{z_R}\right)^2 + 1}{\left(\dfrac{z - z_{f1}}{z_R}\right)^2 + 1}.$$

$$(1.3)$$

Where $\Delta z_f = z_{f2} - z_{f1}$ is the axial shift between the two focus depths, and $z_{f1}$ and $z_{f2}$ are the focus positions of A-line number one and two, respectively. All three parameters $z_{f1}$, $z_R$ and $\Delta z_f$ can be set as fitting parameters. For simplification, one or two of the fitting parameters can be fixed.

Subsequently, a focal position corrected A-line can be calculated and e.g., tissue attenuation coefficients can be determined following, for instance, a procedure described in [6].

To acquire two A-lines with a different focus depth the focus must be changed between the acquisitions of the two A-lines, this is commonly done by axial movement of the sample or of the objective of the system [1-4].

If, for example in human retinal imaging, the eye is accommodating during that focus change, the confocal function cannot reliably be determined. Thus, it is crucial to introduce the focal shift either very fast, e.g., between two consecutive B-scans, or - even better - extract the confocal parameter from one single B-scan.

Especially, the following optional two technical solutions are disclosed:

Solution 1. Adding a dispersive element to the imaging system (or changing the design of an existing lens accordingly), creates different focus positions as a function of wavelength. If the full spectral scan is now divided into subspectra prior to the Fourier transformation, lower resolution A-lines with different focus positions are obtained simultaneously. Dividing those A-lines according to, for instance, Eq. 1.3, and fitting the ratio with the corresponding focal parameters allows for the determination of the wavelength dependent confocal functions.

Solution 2. Alternatively, by adding an active optical element, which can change its refractive power within milliseconds, to the system, two A-scans with a different focus depth can be acquired within milliseconds. Such a fast focus modulation is necessary to reliably determine the confocal function by using the ratio fit method, even during the presence of eye movements and accommodation shifts.

This active element could be either a transmissive element, such as a liquid lens, a motorized lens, or another electro-optical transmissive element, which allows for fast switching of the refractive power. Alternatively, also deformable mirrors could be used in reflection mode to introduce a fast focal shift.

Dividing the A-lines acquired with different focus positions and fitting the ratio with the corresponding focal parameters allows for the determination of the focus depth and Rayleigh length.

The obtained confocal parameters can be used to optimize the focus depth for the desired task or to correct the A-scans for the confocal function. The confocal function correction can be achieved by dividing the original A-scans by the confocal function.

The corrected A-scans can be used to calculate the attenuation coefficient and for the extraction of other quantitative tissue parameter.

In case of solution 1, the A-lines and thus also the B-scans of the subspectra can be corrected for the confocal function individually resulting in different confocal function corrected B-scans for the different wavelengths with a lower resolution than the B-scan of the full spectrum.

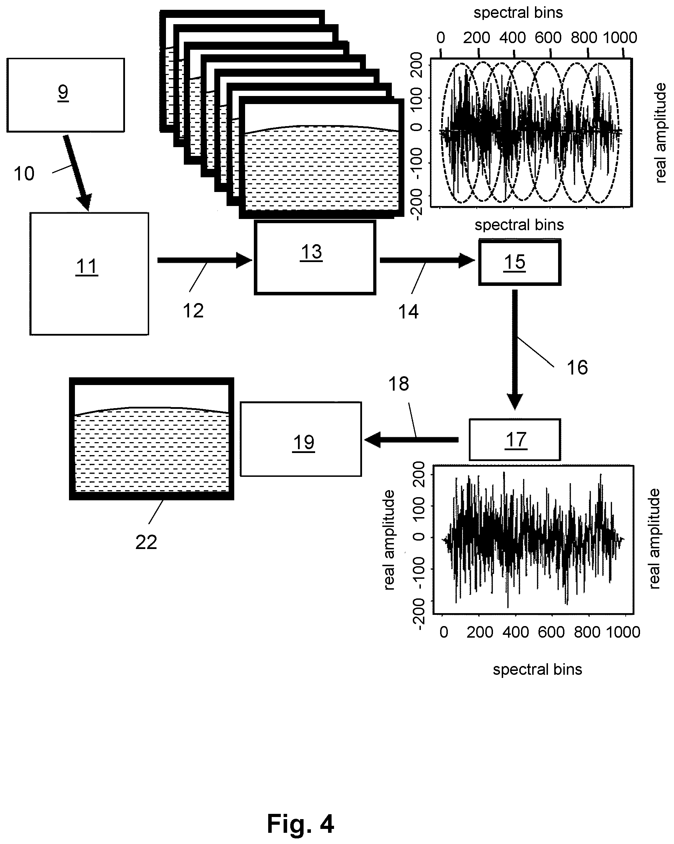

To obtain a high resolution, confocal function corrected B-scan of the full spectrum, the individual confocal function corrected sub-B-scans can be transformed to their spectra using the inverse Fourier transformation, after adding the complex valued subspectra together coherently, the full spectrum can be transformed back to an image using the Fourier transformation (see Figure 4).

Alternatively, the B-scan of the full spectrum can be corrected with a mean confocal function of the subspectra.

While the ratio fitting of A-lines with manually introduced focal shifts has been reported already in literature [1, 2, 3, 4], here, in this description, different methods to obtain focus shifted A-lines without manual adjustment are proposed and disclosed.

1. From one single broad band OCT A-scan, the spectrum can be split into subbands which enables calculation of A-lines for different wavelengths and subsequently different focus depths with the purpose of fitting for the confocal function. The focus parameter can be determined without any additional data acquisition procedure.

2. A fast automatised focus shift between consecutive A- resp. B-scans as described above, could as well allow for accurate extraction of focus parameters, and thus enable the correction of the confocal function with all advantages described above.

The simultaneous/fast acquisition of A-scans with different foci enables to apply the ratio fit method and consequently the determination of the confocal function even to samples or subjects with instable focus location.

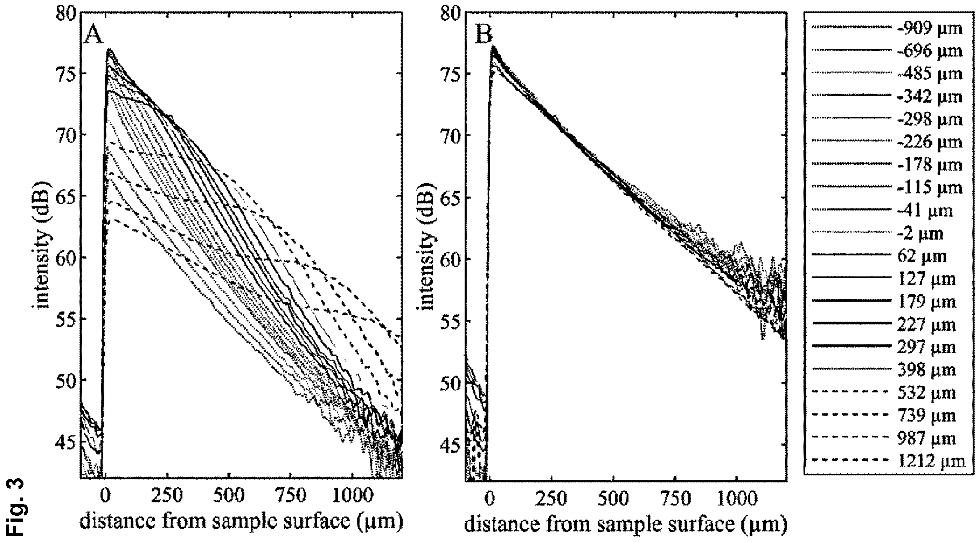

The correction of the confocal function is essential for the determination of absolute tissue scattering data, otherwise the measured results are expected to be erroneous and less reproduceable (see Figure 3).

The following further details and technical features may be optional.

The online display of the focus parameters (mainly focus position) could be used as an online focus control in order to enable optimum image quality during OCT and OCTA data acquisition.

Especially for OCTA data, it is well-known, that the focus position has a significant impact on the reproducible representation of the different capillary vessel plexuses.

This could be realized in form of a software guidance for the user to manually optimize the focal setting or by the implementation of an automized focus adaption or correction algorithm or by means of providing the focal position to the user in a graphical user interface.

It is preferred to reconstruct a high resolution confocal corrected image based on coherent addition of corrected profiles of sub-wavelength bands.

This disclosure especially concerns OCT, confocal function, focus, Rayleigh length, active optical elements, dispersive elements, quantitative OCT, focus control and attenuation coefficients.

In the drawings:

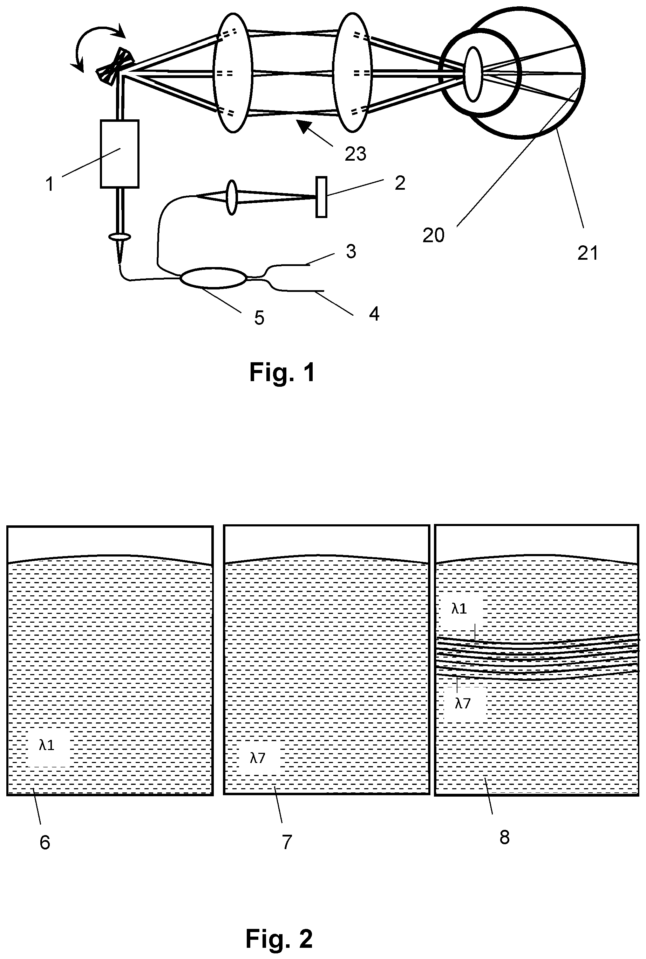

Fig. 1 shows the integration of an active opto-mechanical or electro-optical element for generating a focus shift in the retina for consecutive A- or B-scans. Alternatively, a passive dispersive element, which introduces a chromatic focus shift in the retina, can be added. In this example, the focus shift element is implemented within the stationary beam before scanning, however it could be implemented in the scanning beam as well.

Fig. 2 shows two B-scans obtained from a full-spectrum B-scan by spectral splitting of the frequency data prior to the Fourier transformation. Due to the chromatic aberrations introduced in the imaging system a focus shift can be observed between $\lambda 1$ and $\lambda 7$. The obtained focus planes for the seven different spectral windows can be seen in the right image. From these focus planes a mean focus plane for the whole spectrum can be determined. The scale bar is 200 $\mu$m.

Fig. 3 (from ref. [4]): Left frame (A) shows a series of A-scans acquired at the same location with different focus settings. Right frame (B) shows same data, but now corrected for the confocal function using the ratio fit method for manually shifted A-scans. Note, that over a large range the data follows the Lambert-Beer law of exponential decay. The legend indicates the expected focus depth from the sample surface.

Fig. 4 shows a schematic diagram of the processing steps and

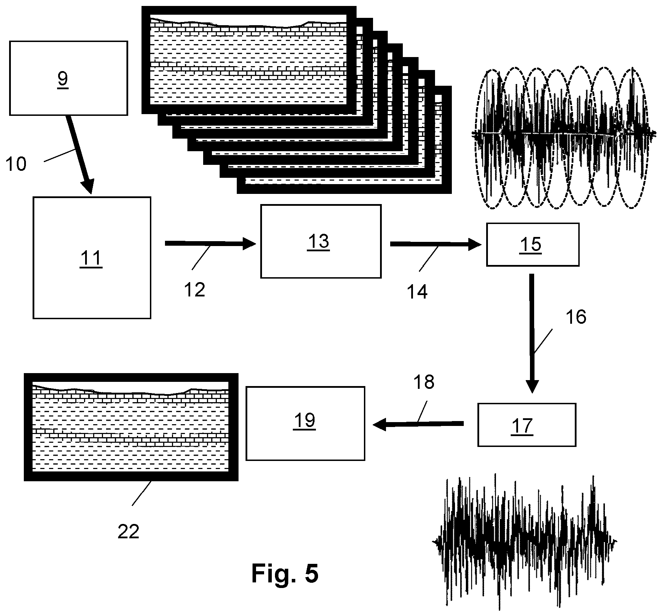

Fig. 5 shows a further schematic diagram of processing steps with invivo-pictures of retinal imaging.

Figure 1 shows a device, comprising at least an interferometer 5, a light source 3, a detection unit 4, a reference mirror 2 and an element 1. The element 1 is a dispersive element or an active lens system.

Fig. 1 as well shows an optical system, especially an OCT imaging system, comprising the said device and further optical means to create an optical path to an eye 21.

The optical system is part of an arrangement, which comprises the optical system and an eye 21.

Figure 1 shows the integration of an active opto-mechanical or electro-optical element 1 for generating a focus shift in the retina 20 for consecutive A- or B-scans.

Alternatively, a passive dispersive element, which introduces a chromatic focus shift in the retina, can be added. In this example, the focus shift element 1 is implemented within the stationary beam before scanning, however it could be implemented in the scanning beam as well.

Figure 2 shows two B-scans 6, 7 obtained from a full-spectrum B-scan by spectral splitting of the frequency data prior to the Fourier transformation.

Due to the chromatic aberrations introduced in the imaging system a focus shift can be observed between $\lambda 1$ and $\lambda 7$.

The obtained focus planes for the seven different spectral windows can be seen in the right image 8. From these focus planes a mean focus plane for the whole spectrum can be determined. The scale bar is 200 $\mu$m.

Figure 3 (from ref. [4]) shows:

(A) Series of A-scans acquired at the same location with different focus settings.

(B) Same data, but now corrected for the confocal function using the ratio fit method for manually shifted A-scans. Note, that over a large range the data follows the Lambert-Beer law of exponential decay. The legend indicates the expected focus depth from the sample surface.

Figure 4 shows a diagram concerning a confocal function corrected high resolution OCT image 22.

The development and evaluation of a method of determining the confocal function by spectral splitting is disclosed in this description.

A proof of principle of the spectral splitting method has been demonstrated in homogeneous intralipid samples of different scatterer concentrations. The feasibility of the method by manually introducing a focus shift has been demonstrated on different samples (also layered samples) and on two human subjects. See publication of Johannes Kübler et al. [4].

Several features described in this disclosure could be implemented in the following matters:

1) Focus depth indicator in the GUI.

2) 3D focus tracking (Add depth tracking and adaptation of the focus for perfect overlay at different timepoints). This requires motorized focus control.

3) Focus depth control in Angio-OCT.

4) For the reproducibility of measurements, which rely on the scattering coefficient of tissue i.e., the intensity of backscattered light, a correction for the confocal function is essential to obtain quantitative results. Of course, this is not the case, if the measured OCT intensity can be normalized to a signal of a structure at very similar z-position and only the ratio of measured intensities is relevant.

In literature, there are several publications (e.g. [5]), which indicate that in glaucoma the tissue degradation of the RNFL leads to a change of the attenuation coefficients, which precedes the thinning of the geometric layer thickness of the RNFL.

If this hypothesis is true, the systematic assessment of reliably measured tissue attenuation coefficients could play an important role in early glaucoma diagnostics.

Since in OCT oximetry signals at different z-positions (before and after transition through a vessel) are evaluated with respect to their spectral range, also here the wavelength dependent position of the focus is of importance.

The hardware of a broad band OCT system optionally could allow the use of such an algorithm as an add-on software tool with additional diagnostic capabilities.

Possible, the existing chromatic focal shift is already sufficient, that such a tool could be used.

A high-resolution OCT system with an extended spectral range may comprise one or several technical features of this description.

Especially, the implementation of additional software modules to exploit the extended spectral range as described is possible.

Fig. 4 and 5 schematically show a method comprising the following steps:

- creating OCT images 6, 7 for different $\lambda$ with different focal positions,
- determining confocal functions,
- creating OCT images for different $\lambda$ with different focal positions and confocal functions,
- correcting for confocal functions to get confocal function corrected OCT images for different $\lambda$,
- applying an inversive Fast Fourier Transformation to get spectra for different $\lambda$,
- adding complex spectra coherently to the spectra for different $\lambda$,
- creating spectrum for full $\lambda$-range,
- applying Fast Fourier Transformation to the spectrum for full $\lambda$-range to create a confocal function corrected high resolution OCT image 22.

The ellipsoids in dashed lines show the spectra for different $\lambda$.

Reference numbers:

1  Dispersive element or active lens system
2  Reference Mirror
3  Light source
4  Detection unit
5  Interferometer
6  B-scan of $\lambda 1$ (OCT image)
7  B-scan of $\lambda 7$ (OCT image)
8  Image of focus planes for seven different spectral windows $\lambda 1$ to $\lambda 7$
9  OCT Images for different $\lambda$ with different focal positions
10  Determine confocal functions
11  OCT Images for different $\lambda$ with different focal positions and confocal functions
12  Correct for confocal functions
13  Confocal function corrected OCT Images for different $\lambda$
14  Inversive FFT
15  Spectra for different $\lambda$
16  Add complex spectra coherently
17  Spectrum for full $\lambda$-range
18  FFT
19  Confocal function corrected high resolution OCT image
20  Retina
21  Eye
22  Confocal function corrected high resolution OCT image
23  Confocal lens arrangement
$\lambda$  wavelength

Abbreviations:

OCTA    Optical coherence tomography angiography
GUI     Graphical user interface
RNFL    retinal nerve fiber layer

References:

[1] Dwork, Nicholas, Gennifer T. Smith, John M. Pauly, and Audrey K. Ellerbee Bowden. 2016. "Automated Estimation of OCT Confocal Function Parameters from two B-Scans." In Conference on Lasers and Electro-Optics, AW1O.4. San Jose, California: Optical Society of America.
[2] Stefan, S., K. S. Jeong, C. Polucha, N. Tapinos, S. A. Toms, and J. Lee. 2018. 'Determination of confocal profile and curved focal plane for OCT mapping of the attenuation coefficient', Biomed Opt Express, 9: 5084-99.
[3] Dwork, N., G. T. Smith, T. Leng, J. M. Pauly, and A. K. Bowden. 2019. 'Automatically Determining the Confocal Parameters From OCT B-Scans for Quantification of the Attenuation Coefficients', IEEE Trans Med Imaging, 38: 261-68.
[4] Kübler, J., V. S. Zoutenbier, A. Amelink, J. Fischer and J. F. de Boer (2021). "Investigation of methods to extract confocal function parameters for the depth resolved determination of attenuation coefficients using OCT in intralipid samples, titanium oxide phantoms, and in vivo human retinas." Biomedical Optics Express 12(11).
[5] Vermeer, K. A., J. van der Schoot, H. G. Lemij and J. F. de Boer (2012). "RPE-normalized RNFL attenuation coefficient maps derived from volumetric OCT imaging for glaucoma assessment." Invest Ophthalmol Vis Sci 53(10): 6102-6108.
[6] Vermeer, K. A., J. Mo, J. J. Weda, H. G. Lemij and J. F. de Boer (2013). "Depth-resolved model-based reconstruction of attenuation coefficients in optical coherence tomography." Biomed Opt Express 5(1): 322-337.

**Claims**

1.  OCT imaging system to perform a method for confocal function determination in the OCT imaging system, comprising a device, which is configured to determine the confocal function, wherein the device comprises at least an interferometer (5), a light source (3) for emitting light of different wavelengths, a detection unit (4), and wherein the OCT imaging system comprises a confocal lens arrangement (23) to create retinal imaging, wherein the confocal function of the imaging system is

$$h(z) = \left[ \left( \frac{z - z_f}{z_R} \right)^2 + 1 \right]^{-1}$$

with focus position $z_f$ and Rayleigh length $z_R$, wherein the device is configured to determine the confocal function by exploiting the common characteristics of two A-lines of the same sample location acquired with different focus positions, by taking the ratio of the two A-lines (I (z)) acquired with a focus offset and fitting the following equation

$$\frac{I_1(z)}{I_2(z)} = \frac{h_1(z)}{h_2(z)} = \frac{\left( \frac{z - (z_{f1} + \Delta z_f)}{z_R} \right)^2 + 1}{\left( \frac{z - z_{f1}}{z_R} \right)^2 + 1}$$

to the ratio of the two A-lines to determine the focus position $z_f$ and Rayleigh length $z_R$, where $\Delta z_f = z_{f2} - z_{f1}$ is the axial shift between the two focus positions, and $z_{f1}$ and $z_{f2}$ are the focus positions of the respective A-lines, **characterized in that** the device is configured to change the focus position between the acquisitions of the two A-lines to acquire the two A-lines with a different focus position, wherein the device comprises an active optical element (1) which can change its refractive power, to obtain focus shifted A-lines without manual adjustment, and/ or **characterized in that** the device comprises a dispersive element (1) configured to create the different focus positions as a function of wavelength ($\lambda$) of the light source (3) to obtain focus shifted A-lines without manual adjustment.

2. System according to claim 1, **characterized in that**, the device applies a Fourier Transformation to a spectrum and creates a confocal function corrected high resolution OCT image (22).

3. Method for confocal function determination in the OCT imaging system using the system according to one of the preceding claims, comprising at least:

   - determining a confocal function.

4. Method according to claim 3, comprising the step of reconstruction of a high resolution confocal corrected image (22).

5. Method according to claim 3 or 4, **characterized in that** the reconstruction of the high resolution confocal corrected image (22) is based on coherent addition of corrected profiles of sub-wavelength bands.


**Patentansprüche**

1. OCT-Bildgebungssystem zum Durchführen eines Verfahrens zur konfokalen Funktionsermittlung in dem OCT-Bildgebungssystem, das eine Vorrichtung umfasst, die zum Ermitteln der konfokalen Funktion konfiguriert ist, wobei die Vorrichtung mindestens ein Interferometer (5), eine Lichtquelle (3) zum Emittieren von Licht unterschiedlicher Wellenlängen und eine Erkennungseinheit (4) umfasst, und wobei das OCT-Bildgebungssystem eine konfokale Linsenanordnung (23) umfasst, um eine Netzhautbildgebung zu erzeugen, wobei die konfokale Funktion des

Bildgebungssystems lautet:

$$h(z) = \left[\left(\frac{z - z_f}{z_R}\right)^2 + 1\right]^{-1}$$

mit einer Fokusposition $z_f$ und einer Rayleigh-Länge $z_R$, wobei die Vorrichtung konfiguriert ist zum Ermitteln der konfokalen Funktion, indem die gemeinsamen Eigenschaften von zwei A-Linien desselben Probenortes ausgenutzt werden, die mit unterschiedlichen Fokuspositionen erfasst wurden, indem das Verhältnis der zwei A-Linien (I(z)) übernommen wird, die mit einem Fokusversatz erfasst wurden, und die folgende Gleichung

$$\frac{I_1(z)}{I_2(z)} = \frac{h_1(z)}{h_2(z)} = \frac{\left(\frac{z - (z_{f1} + \Delta z_f)}{z_R}\right)^2 + 1}{\left(\frac{z - z_{f1}}{z_R}\right)^2 + 1}$$

auf das Verhältnis der beiden A-Linien angepasst wird, um die Fokusposition $z_f$ und die Rayleigh-Länge $z_R$ zu ermitteln, wobei $\Delta z_f = z_{f2} - z_{f1}$ die axiale Verschiebung zwischen den beiden Fokuspositionen ist und $z_{f1}$ und $z_{f2}$ die Fokuspositionen der jeweiligen A-Linien sind, **dadurch gekennzeichnet, dass** die Vorrichtung konfiguriert ist zum Ändern der Fokusposition zwischen den Erfassungen der beiden A-Linien, um die beiden A-Linien mit unterschiedlichen Fokuspositionen zu erfassen, wobei die Vorrichtung ein aktives optisches Element (1) umfasst, das seine Brechkraft ändern kann, um fokusverschobene A-Linien ohne eine manuelle Anpassung zu erhalten, und/oder **dadurch gekennzeichnet, dass** die Vorrichtung ein dispersives Element (1) umfasst, das konfiguriert ist zum Erzeugen der unterschiedlichen Fokuspositionen als eine Funktion der Wellenlänge ($\lambda$) der Lichtquelle (3), um fokusverschobene A-Linien ohne eine manuelle Anpassung zu erhalten.

2. System nach Anspruch 1, **dadurch gekennzeichnet, dass** die Vorrichtung eine Fourier-Transformation auf ein Spektrum anwendet und ein konfokales funktionskorrigiertes hochauflösendes OCT-Bild (22) erzeugt.

3. Verfahren für eine konfokale Funktionsermittlung in dem OCT-Bildgebungssystem unter Verwendung des Systems nach einem der vorhergehenden Ansprüche, mindestens umfassend:

   - Ermitteln einer konfokalen Funktion.

4. Verfahren nach Anspruch 3, das den Schritt einer Rekonstruktion eines hochauflösenden konfokalen korrigierten Bildes (22) umfasst.

5. Verfahren nach Anspruch 3 oder 4, **dadurch gekennzeichnet, dass** die Rekonstruktion des hochauflösenden konfokalen korrigierten Bildes (22) auf einer kohärenten Addition korrigierter Profile von Unter-Wellenlängen-Bändern basiert ist.

**Revendications**

1. Système d'imagerie OCT pour réaliser un procédé de détermination de fonction confocale dans le système d'imagerie OCT, comprenant un dispositif, qui est configuré pour déterminer la fonction confocale, le dispositif comprenant au moins un interféromètre (5), une source lumineuse (3) pour émettre de la lumière de différentes longueurs d'onde, une unité de détection (4), et le système d'imagerie OCT comprenant un agencement de lentilles confocales (23) pour créer une imagerie rétinienne, la fonction confocale du système d'imagerie étant

$$h(z) = \left[\left(\frac{z - z_f}{z_R}\right)^2 + 1\right]^{-1}$$

avec une position focale $z_f$ et la longueur de Rayleigh $z_R$, le dispositif étant configuré pour déterminer la fonction confocale en exploitant les caractéristiques communes de deux lignes A du même emplacement d'échantillon acquises avec des positions focales différentes, en prenant le rapport des deux lignes A (I(z)) acquises avec un décalage de focalisation et en ajustant l'équation suivante

$$\frac{I_1(z)}{I_2(z)} = \frac{h_1(z)}{h_2(z)} = \frac{\left(\dfrac{z - (z_{f1} + \Delta z_f)}{z_R}\right)^2 + 1}{\left(\dfrac{z - z_{f1}}{z_R}\right)^2 + 1}$$

au rapport des deux lignes A pour déterminer la position focale $z_f$ et la longueur de Rayleigh $z_R$, $\Delta z_f = z_{f2} - z_{f1}$ étant le décalage axial entre les deux positions focales, et $z_{f1}$ et $z_{f2}$ étant les positions focales des lignes A respectives, **caractérisé en ce que** le dispositif est configuré pour modifier la position focale entre les acquisitions des deux lignes A pour acquérir les deux lignes A avec une position focale différente, le dispositif comprenant un élément optique actif (1) pouvant changer sa puissance de réfraction, pour obtenir des lignes A décalées de focalisation sans réglage manuel, et/ ou **caractérisé en ce que** le dispositif comprend un élément dispersif (1) configuré pour créer les différentes positions focales en fonction de la longueur d'onde ($\lambda$) de la source de lumière (3) pour obtenir des lignes A décalées de focalisation sans ajustement manuel.

2. Système selon la revendication 1, **caractérisé en ce que** le dispositif applique une transformation de Fourier à un spectre et crée une image OCT à haute résolution corrigée par fonction confocale (22).

3. Procédé de détermination de fonction confocale dans le système d'imagerie OCT utilisant le système selon l'une des revendications précédentes, comprenant au moins :

    - la détermination d'une fonction confocale.

4. Procédé selon la revendication 3, comprenant l'étape de reconstruction d'une image corrigée confocale à haute résolution (22).

5. Procédé selon la revendication 3 ou 4, **caractérisé en ce que** la reconstruction de l'image corrigée confocale à haute résolution (22) est basée sur l'addition cohérente de profils corrigés de bandes de longueurs d'onde inférieures.

**Fig. 1**

**Fig. 2**

Fig. 3

**Fig. 4**

**Fig. 5**

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Non-patent literature cited in the description

- Automatically Determining the Confocal Parameters from OCT B-Scans for Quantification of the Attenuation Coefficients. **DWORKS NICHOLAS et al.** IEE TRANSACTIONS IN MEDICAL IMAGING. IEEE, 01 January 2019, vol. 38, 261-268
- Automated Estimation of OCT Confocal Function Parameters from two B-Scans.. **DWORK, NICHOLAS ; GENNIFER T. SMITH ; JOHN M. PAULY ; AUDREY K. ELLERBEE BOWDEN**. Conference on Lasers and Electro-Optics, AW1O.4.. Optical Society of America, 2016
- **STEFAN, S. ; K. S. JEONG ; C. POLUCHA ; N. TAPINOS ; S. A. TOMS ; J. LEE.** Determination of confocal profile and curved focal plane for OCT mapping of the attenuation coefficient. *Biomed Opt Express*, 2018, vol. 9, 5084-99
- **DWORK, N. ; G. T. SMITH ; T. LENG ; J. M. PAULY ; A. K. BOWDEN.** Automatically Determining the Confocal Parameters From OCT B-Scans for Quantification of the Attenuation Coefficients. *IEEE Trans Med Imaging*, 2019, vol. 38, 261-68

- **KÜBLER, J. ; V. S. ZOUTENBIER ; A. AMELINK ; J. FISCHER ; J. F. DE BOER**. Investigation of methods to extract confocal function parameters for the depth resolved determination of attenuation coefficients using OCT in intralipid samples, titanium oxide phantoms, and in vivo human retinas.. *Biomedical Optics Express*, 2021, vol. 12 (11)
- **VERMEER, K. A. ; J. VAN DER SCHOOT ; H. G. LEMIJ ; J. F. DE BOER**. RPE-normalized RNFL attenuation coefficient maps derived from volumetric OCT imaging for glaucoma assessment.. *Invest Ophthalmol Vis Sci*, 2012, vol. 53 (10), 6102-6108
- **VERMEER, K. A. ; J. MO ; J. J. WEDA ; H. G. LEMIJ ; J. F. DE BOER**. Depth-resolved model-based reconstruction of attenuation coefficients in optical coherence tomography.. *Biomed Opt Express*, 2013, vol. 5 (1), 322-337